# FASCICULE DE BREVET EUROPEEN

(11) **EP 1 635 715 B1**
(45) Date de publication et mention de la délivrance du brevet: **07.10.2015**
(21) Numéro de dépôt: 04742888.3
(22) Date de dépôt: 18.06.2004
(51) Int. Cl.: A61B 17/15, A61B 17/17

(54) **DISPOSITIF DE GUIDAGE DE COUPE OSSEUSE**
FÜHRUNGSVORRICHTUNG FÜR KNOCHENRESEKTION
GUIDING DEVICE FOR BONE CUTTING

(30) Priorité: 18.06.2003 FR 0307346
(43) Date de publication de la demande: 22.03.2006
(73) Titulaire: Perception Raisonnement Action En Medecine, 38700 La Tronche (FR)
(72) Inventeur: PLASKOS, Christopher, New York, NY (US); LAVALLEE, Stéphane, F-38410 SAINT MARTIN D'URIAGE (FR); CHAMPLEBOUX, Guillaume, F-38500 VOIRON (FR)
(74) Mandataire: de Beaumont, Michel
(86) Numéro de dépôt international: PCT/FR2004/050227
(87) Numéro de publication internationale: WO 2004/112620

(56) Documents cités:
- WO-A-98/32384
- WO-A1-00/00093
- US-A- 4 457 307
- US-A- 5 653 714
- US-A- 5 860 981

## Description

La présente invention concerne un dispositif de guidage de coupe osseuse.

Certaines opérations chirurgicales, par exemple l'arthroplastie de la hanche ou du genou, requièrent la réalisation d'au moins une coupe osseuse au niveau de la tête d'un os long pour la mise en place d'une prothèse. La coupe osseuse est réalisée au moyen d'un outil de coupe, par exemple une fraiseuse ou une scie oscillante, maintenu en position par un dispositif de guidage de coupe fixé à l'os. Généralement, plusieurs coupes osseuses drivent être réalisées successivement dans des plans distincts au niveau d'une même tête d'os. Il est alors nécessaire de déterminer avec précision les positions relatives entre les plans de coupe, tout en assurant un état de surface de qualité suffisant pour chaque plan de coupe.

Le brevet américain US 4457307 décrit un dispositif de guidage d'un outil de coupe pour la réalisation de coupes successives selon différents plans de coupe lors de la mise en place d'une prothèse du genou. Le dispositif comporte une base destinée à être fixée sur la diaphyse du fémur. Un guide de coupe est monté pivotant sur des bras, eux-mêmes montés pivotants sur la base. Le guide peut alors être déplacé par rapport à la base pour définir les plans de coupe. Une scie est maintenue par le guide lors de la réalisation des coupes osseuses. Le préambule de la revendication 1 est basé sur ce dispositif. Un inconvénient d'un tel dispositif est qu'il est relativement volumineux.

Le brevet américain US 5653714 décrit un dispositif de guidage d'un outil de coupe osseuse pour la réalisation de coupes osseuses successives selon des plans de coupe distincts présentant un encombrement réduit.

La présente invention vise à obtenir une alternative de dispositif de guidage de coupe adapté à la réalisation d'une coupe osseuse, ou de plusieurs coupes osseuses successives selon différents plans de coupe, présentant un encombrement réduit.

La présente invention vise également à obtenir un dispositif de guide de coupe ayant une structure et une cinématique particulièrement simples.

Pour atteindre ces objets, la présente invention prévoit un dispositif de guidage d'un outil de coupe adapté à couper des portions osseuses au niveau d'une tête d'un os, comprenant une embase destinée à être fixée au niveau de ladite tête ; un moyen d'ajustement de la position d'un premier axe de rotation par rapport à ladite embase ; un bras, une extrémité dudit bras étant montée pivotante sur ladite embase selon le premier axe de rotation ; et un guide destiné à porter l'outil et monté pivotant sur le bras selon un second axe de rotation (Δ).

Selon un mode de réalisation de l'invention, le dispositif de guidage comprend deux embases destinées à être fixées au niveau de ladite tête de façon sensiblement opposée par rapport à ladite tête ; un moyen d'ajustement de la position du premier axe de rotation par rapport aux embases ; deux bras, une extrémité de chaque bras étant montée pivotante sur une embase selon le premier axe de rotation ; et un guide s'étendant sensiblement transversalement à ladite tête, et monté pivotant à deux extrémités sur les bras selon un second axe de rotation, l'angle de débattement maximal du guide autour du premier axe de rotation étant supérieur à 100 degrés.

Selon un mode de réalisation de l'invention, l'un des bras est relié à une embase par une liaison à pivot, le système d'ajustement étant disposé entre ladite embase et ladite liaison à pivot et étant adapté à modifier la position de ladite liaison à pivot par rapport à ladite embase selon deux directions sensiblement tangentes à ladite tête.

Selon l'invention, le dispositif comprend un premier actionneur adapté à entraîner en rotation au moins un bras autour du premier axe de rotation et un second actionneur adapté à entraîner en rotation le guide autour du second axe de rotation.

Selon un mode de réalisation de l'invention, le dispositif comprend un élément de liaison reliant les deux embases en entourant au moins partiellement ladite tête.

Selon un mode de réalisation de l'invention, le moyen d'ajustement est disposé entre l'une des embases et l'élément de liaison.

Selon un mode de réalisation de l'invention, le dispositif comprend un moyen de déplacement de l'une des embases par rapport à l'élément de liaison dans une direction transversale à ladite tête.

Selon un mode de réalisation de l'invention, chaque embase comprend au moins un pic destiné à venir en contact avec ladite tête.

Selon un mode de réalisation de l'invention, le guide comprend au moins une ouverture pour guider l'outil de coupe.

Selon un mode de réalisation de l'invention, l'outil de coupe est une fraise.

Selon un mode de réalisation de l'invention, l'outil de coupe est une scie oscillante

Ces objets, caractéristiques et avantages, ainsi que d'autres de la présente invention seront exposés en détail dans la description suivante d'exemples de réalisation particuliers faite à titre non-limitatif en relation avec les figures jointes parmi lesquelles :
la figure 1 représente une vue en perspective d'un premier exemple de réalisation du dispositif de guidage de coupe selon l'invention ;
la figure 2 représente une vue de dessus du dispositif de la figure 1 ;
la figure 3 représente, de façon schématique, des vues de côté du dispositif de la figure 1 lors de la réalisation de plusieurs plans de coupe ;
la figure 4 représente des vues analogues à la figure 3 pour une variante d'utilisation du dispositif de la figure 1 ;
la figure 5 représente une vue en perspective de certains éléments d'un second exemple de réalisation du dispositif de guidage selon l'invention ;
la figure 6 représente une vue en perspective de la totalité du dispositif de la figure 5 ;
la figure 7 illustre schématiquement le principe du réglage de la position du dispositif selon l'invention ; et
la figure 8 représente une vue de dessus d'un troisième exemple de réalisation du dispositif dé guidage de coupe selon l'invention.

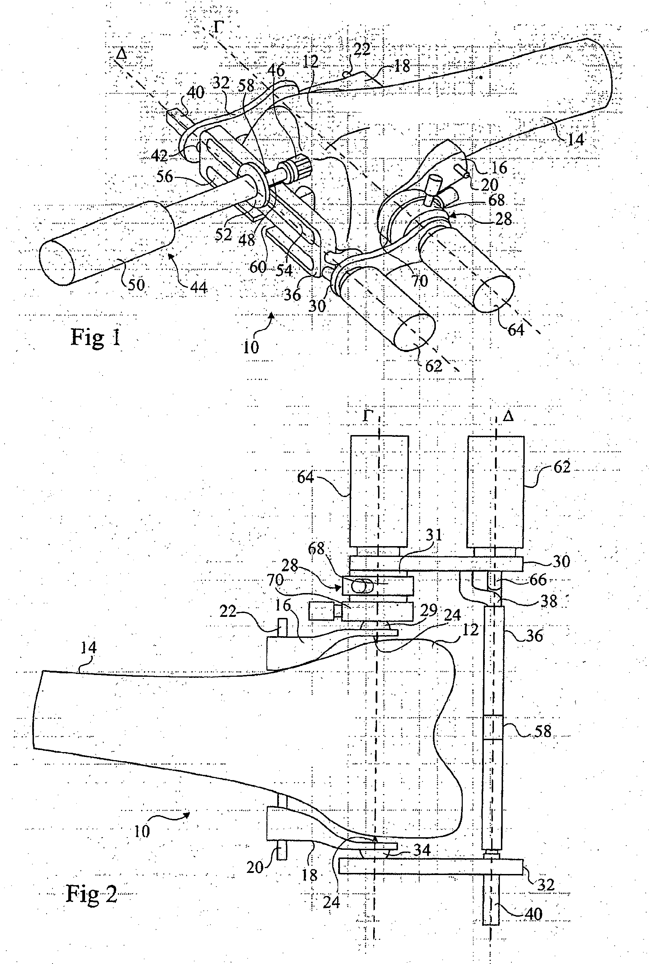

Comme cela est représenté aux figures 1 et 2, le dispositif de guidage de coupe 10 selon l'invention est disposé au niveau de la tête 12 d'un os long 14. Il s'agit par exemple du fémur, du tibia, de l'humérus, etc. Le dispositif 10 comprend deux embases 16, 18 fixées à la tête 12 de l'os 14 par l'intermédiaire de chevilles 20, 22. Chaque embase 16, 18 comprend un pic 24 pour la mise en place de l'embase 16, 18 sur la tête 12 avant la fixation définitive de l'embase 16, 18 au moyen de la cheville 20, 22 associée.

Un système d'ajustement 28 est monté sur l'embase 16 par l'intermédiaire d'un joint sphérique 29. Un bras 30 est monté pivotant sur le système d'ajustement 28 par l'intermédiaire d'une liaison à pivot 31. Un bras 32 est monté mobile sur l'embase 18 par l'intermédiaire d'un joint sphérique 34.

Le dispositif 10 comprend un guide de coupe 36 qui s'étend entre les bras 30, 36. Une extrémité du guide de coupe 36 est montée pivotante sur un levier 38 fixé au bras 30 selon un axe de rotation Δ. L'extrémité opposée 40 du guide de coupe 36 est montée pivotante dans une ouverture 42 du bras 32. La liaison entre les bras 30, 32 imposée par le guide de coupe 36 limite sensiblement le mouvement des bras 30, 32 par rapport aux embases 16, 18 à un mouvement de rotation autour d'un axe de rotation Γ.

Le guide de coupe 36 est destiné à recevoir un outil de coupe 44, représenté uniquement en figure 1. Dans le présent exemple de réalisation, l'outil de coupe 44 est une fraiseuse comprenant une fraise 46 agencée à l'extrémité d'un arbre 48 entraîné par un moteur électrique 50. L'outil de coupe 44 comprend également une surface d'appui 52 destinée à venir en butée contre le guide 36 et qui s'étend dans un plan sensiblement perpendiculaire à l'axe de l'arbre 48. La surface d'appui 52 maintient l'arbre 48 dans une direction sensiblement perpendiculaire à l'axe Δ lorsqu'un manipulateur applique l'outil 44 contre le guide 36. Le guide 36 est traversé par deux rainures 54, 56 sensiblement droites et parallèles qui s'étendent selon l'axe Δ dans lesquelles peut coulisser l'arbre moteur 48. Deux encoches 58, 60 sont prévues pour permettre l'insertion de l'arbre moteur 48 dans l'une des rainures 54, 56. L'outil de coupe 44 comprend un moyen (non représenté) de réglage de la distance séparant la fraise 46 de la surface d'appui 52.

Deux moteurs électriques 62, 64 sont portés par le bras 30. Les moyens d'alimentation et de commande des moteurs 62, 64 ne sont pas représentés. Le moteur électrique 62 est adapté à entraîner en rotation le guide 36 autour de l'axe Δ. Pour ce faire, l'arbre moteur du moteur électrique 62 est relié au guide 36 par l'intermédiaire d'un bras coudé 66. Le moteur électrique 64 est destiné à entraîner en rotation le bras 30 autour de l'axe de rotation Γ. Les moteurs 62, 64 peuvent être commandés par un calculateur non représenté.

Le système d'ajustement 28 permet de régler avec précision la position de la liaison de pivot 31 par rapport à l'embase 16 afin d'autoriser, lors du montage du dispositif de guidage 10, la rotation des bras 30, 32 autour de l'axe de rotation Γ. Le système d'ajustement 28 comprend deux moyens de réglage 68, 70 qui permettent de modifier la position de la liaison de pivot 31 par rapport à la tête 12 de l'os 14 selon deux directions sensiblement perpendiculaires. Selon le présent exemple de réalisation, les moyens de réglage 68, 70 sont constitués de vis réglables actionnées manuellement par un utilisateur. Les matériaux utilisés pour la réalisation des pièces constituant le dispositif de guidage 10 sont compatibles avec les contraintes de stérilité propres au domaine médical.

Le montage du dispositif 10 selon le premier exemple de réalisation sur la tête 12 de l'os 14 est le suivant. Un premier ensemble formé par l'embase 16, le bras 30 et le guide de coupe 36, et un second ensemble formé par l'embase 18 et le bras 32 sont disposés approximativement au niveau de la tête 12 de façon sensiblement opposée par rapport à la tête 12. Les embases 16, 18 sont rapprochées de façon à insérer l'extrémité 40 du guide 36 dans l'ouverture 42 du bras 32. Les joints sphériques 29, 34 compensent les jeux de montage lors de l'assemblage du guide de coupe 36 avec le bras 32. La position du bras 30 par rapport à l'embase 16 est alors réglée avec précision par l'intermédiaire du système d'ajustement 28 pour définir avec précision la position de l'axe de rotation Γ. Pour simplifier la cinématique du dispositif de guidage 10, les dimensions des bras 30, 32 sont définies de façon que les axes de rotation Γ et Δ soient parallèles.

Le fonctionnement du dispositif 10 une fois fixé à la tête 12 de l'os 14 est le suivant. L'actionnement du moteur 64 permet de déplacer globalement le guide de coupe 36 par rapport à la tête 12 de l'os 14 autour de l'axe Γ. L'actionnement du moteur 62 permet d'incliner le guide de coupe 36 autour de l'axe Δ ce qui fixe la direction de l'arbre moteur 48 de l'outil de coupe 44 une fois ce dernier disposé au niveau du guide de coupe 36. La distance séparant la fraise 46 et la surface d'appui 52 permet alors de choisir la position du plan de coupe parmi une famille de plans de coupe parallèles.

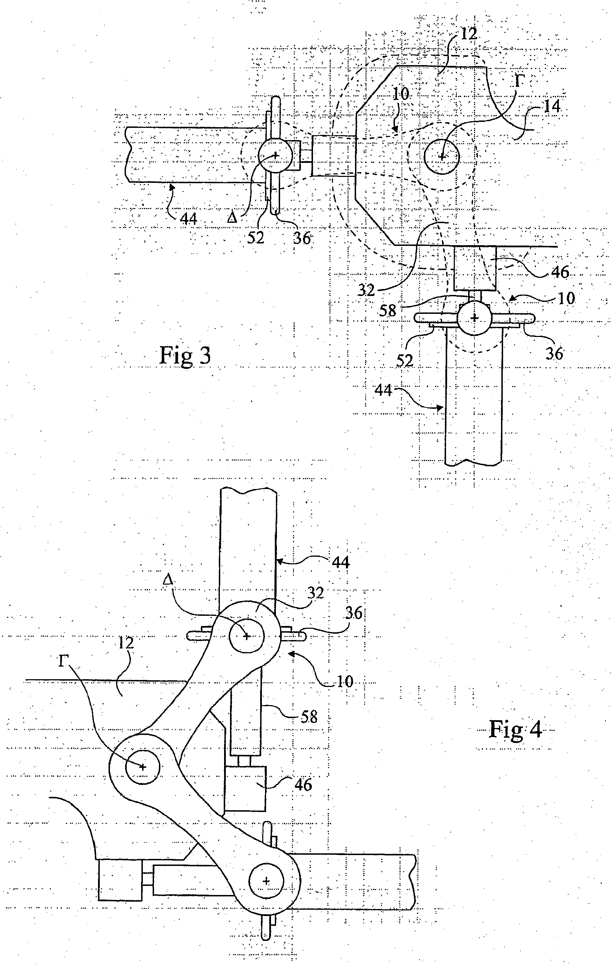

La figure 3 représente, de façon schématique, le dispositif 10 selon l'invention équipé de l'outil de coupe 44 lors de la réalisation de deux coupes osseuses selon deux plans de coupe distincts. En figure 3, le contour de la tête 12 de l'os 14 est représenté en traits mixtes en l'absence de coupes osseuses et en trait plein une fois les coupes réalisées. Pour une meilleure visibilité, le bras 32 est représenté en pointillés. Selon l'exemple de réalisation de coupes osseuses illustré en figure 3, le fraisage est réalisé frontalement. L'axe de la fraise 46 est alors disposé sensiblement perpendiculairement au plan de coupe.

Selon l'exemple de réalisation de coupes osseuses illustré en figure 4, le fraisage est réalisé latéralement. L'axe de la fraise 46 est alors disposé sensiblement parallèlement au plan de coupe.

Comme cela apparaît sur les figures 3 et 4, les différentes coupes osseuses sont réalisées selon des plans de coupe qui se coupent selon des droites parallèles à une direction appelée axe de la prothèse. Il suffit donc de prévoir un mouvement de rotation autour de l'axe de prothèse pour positionner tous les plans de coupe. Le dispositif selon la présente invention permet de positionner le guide de coupe 36. Pour ajuster la position du guide de coupe 36, la présente invention prévoit deux mécanismes de réglage. Le premier mécanisme de réglage est constitué du système d'ajustement 28 à deux degrés de liberté qui permet de positionner l'axe de rotation Γ parallèle à l'axe de la prothèse. Le second mécanisme de réglage est constitué des deux moteurs 62, 64 et permet, par le contrôle de deux degrés de liberté, de positionner le guide de coupe 36. Plus précisément, les deux moteurs 62, 64 entraînent en rotation des éléments du dispositif 10 selon deux axes de rotation parallèles Γ, Δ. L'axe de rotation Γ est prévu au niveau de la tête 12. L'axe de rotation Δ est en rotation autour de l'axe de rotation Γ. Un tel dispositif 10 à deux degrés de liberté permet d'atteindre toutes les configurations souhaitées de plans de coupe de la tête dès que l'axe de rotation Γ est correctement positionné.

Le dispositif 10 selon la présente invention permet l'obtention d'angles de débattement importants du guide de coupe 36 autour de l'axe de rotation Γ. A titre d'exemple, on peut atteindre des débattements supérieurs à 100 degrés autour de l'axe de rotation Γ, l'inclinaison du guide de coupe 36 autour de l'axe Δ étant suffisante pour atteindre toutes les positions de plan de coupe souhaitées. L'inclinaison du guide de coupe 36 par rapport à l'axe Δ permet notamment de choisir un fraisage latéral ou frontal.

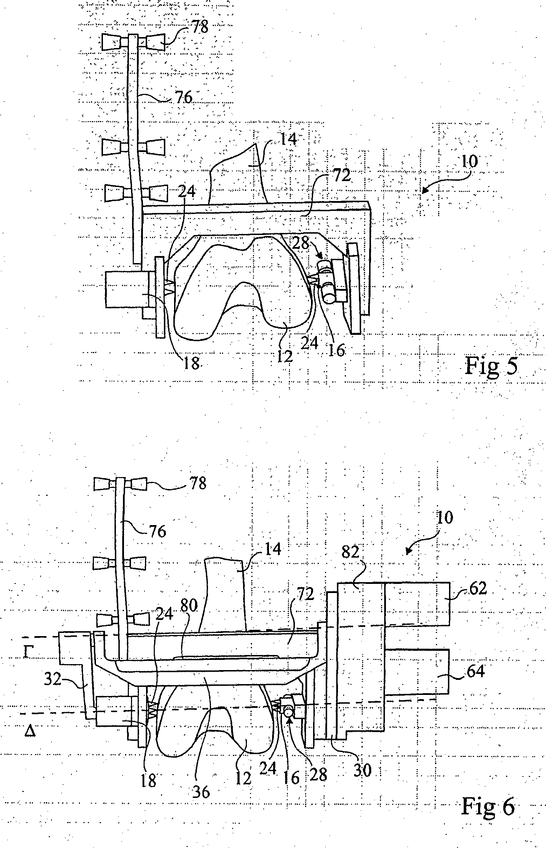

Les figures 5 et 6 représentent un second exemple de réalisation du dispositif de guidage 10 selon l'invention. Les éléments communs avec le premier exemple de réalisation sont indiqués par des références identiques. Selon le second exemple de réalisation de l'invention, les embases 16, 18 sont reliées par un élément de liaison 72 qui contourne la tête 12 de l'os 14 depuis une embase 16 jusqu'à l'autre embase 18. A titre d'exemple, l'élément de liaison 72 est en forme de "U", de "V", de "C", etc. Le système d'ajustement 28 est disposé entre l'embase 16 et l'élément de liaison 72. L'embase 18 peut être déplacée, par exemple par un mécanisme à vis, par rapport à l'élément de liaison 72 de façon à être rapprochée ou éloignée de l'autre embase 16. Chaque embase 16, 18 comporte au moins trois pics 24.

Un corps rigide 76, fixé à l'élément de liaison 72, porte des facettes rétro-refléchissantes 78 (six facettes rétro-réfléchissantes étant représentées sur les figures 5 et 6). Le corps rigide 76 fait partie d'un système de localisation (non représenté) adapté à déterminer la position de l'élément de liaison 72. Le système de localisation est par exemple du type comprenant une source émettant un rayonnement infrarouge et plusieurs capteurs mesurant le rayonnement infrarouge réfléchi par les facettes rétro-réfléchissantes 78. Il est à noter que tout système de localisation de l'élément de liaison 72 peut être utilisé. A titre d'exemple, le système de localisation peut être à base de technologie optique (tel le système POLARIS de la société NDI, Toronto, Canada), à base de technologie magnétique (tel que le système Fastrack de la société Polhemus Inc, Etats-Unis), ou à base de technologie ultrasonore (produit de la société Zebris, Allemagne).

La figure 6 représente la structure complète du dispositif de guidage de coupe 10 selon le second exemple de réalisation de l'invention. Le dispositif 10 est destiné à être utilisé avec un outil de coupe (non représenté) du type scie oscillante. Le guide de coupe 36 comprend alors une fente plane 80 délimitée par deux parois planes supérieures et inférieures et dans laquelle est insérée la scie oscillante. Selon la disposition du guide de coupe 36, la paroi supérieure ou la paroi inférieure est confondue avec le plan de coupe. Comme pour le premier exemple de réalisation, le guide 36 est monté pivotant autour de l'axe de rotation Δ sur les bras 32, 30 eux-mêmes montés pivotant sur les embases 16, 18 autour de l'axe de rotation Γ. Les moteurs 62, 64 sont disposés sur un socle 82 monté sur le bras 30. Le moteur 64 entraîne en rotation le bras 30 par rapport à l' embase 16 autour de l'axe Γ et le moteur 62 entraîne en rotation le guide 36 par rapport au bras 30 autour de l'axe Δ. Il est avantageux de prévoir un montage simple et rapide du socle 82 sur le bras 30. Pour ce faire, le bras 30 peut comporter au niveau d'un emplacement ou de plusieurs emplacements une surface à trois rainures (non représentées) réparties en étoile et recevant des billes agencées au niveau de la plaque 82. Le bras 30 comporte en outre un filetage disposé au centre de la répartition étoilée de rainures. Le montage du socle 82 sur le bras est alors assuré au moyen d'une vis prenant en maintenant en appui le bloc 82 contre le bras 30.

Le montage du dispositif 10 selon le second exemple de réalisation de l'invention est effectué en écartant suffisamment les embases 16, 18 l'une de l'autre de façon à insérer la tête 12 entre les deux embases 16, 18 approximativement selon la position finale souhaitée. On rapproche alors les embases 16, 18 l'une de l'autre de façon que les pics 24 des embases 16, 18 viennent au contact de la tête 12. Le système d'ajustement 28 permet alors de régler avec précision la position relative entre l'élément de liaison 72 et l'embase 16 pour définir la position de l'axe de rotation Γ. La fixation définitive des embases 16, 18 à la tête 12 peut être obtenue au moyen de broches ou de vis de fixation non représentées. Le réglage de la position de l'axe de rotation Γ par le système d'ajustement 28 peut être assisté par ordinateur. En effet, on peut prévoir d'afficher sur un écran de visualisation la position théorique dé l'axe de rotation Γ fournie à partir d'une modélisation de l'os 14 et la position réelle de l'axe de rotation Γ obtenue à partir de la position de l'élément de liaison 72 fournie à partir du corps rigide 76.

La commande à appliquer aux moteurs 62, 64 lors du positionnement du dispositif 10 pour la réalisation des coupes osseuses peut être fournie par un calculateur à partir de la position de l'élément de fixation 72 fournie à partir du corps rigide 76, d'une position initiale du guide de coupe 36 obtenue en appliquant temporairement un système de repérage de position au niveau de la fente 80 du guide de coupe 36 et de paramètres de fonctionnement des moteurs 62, 64.

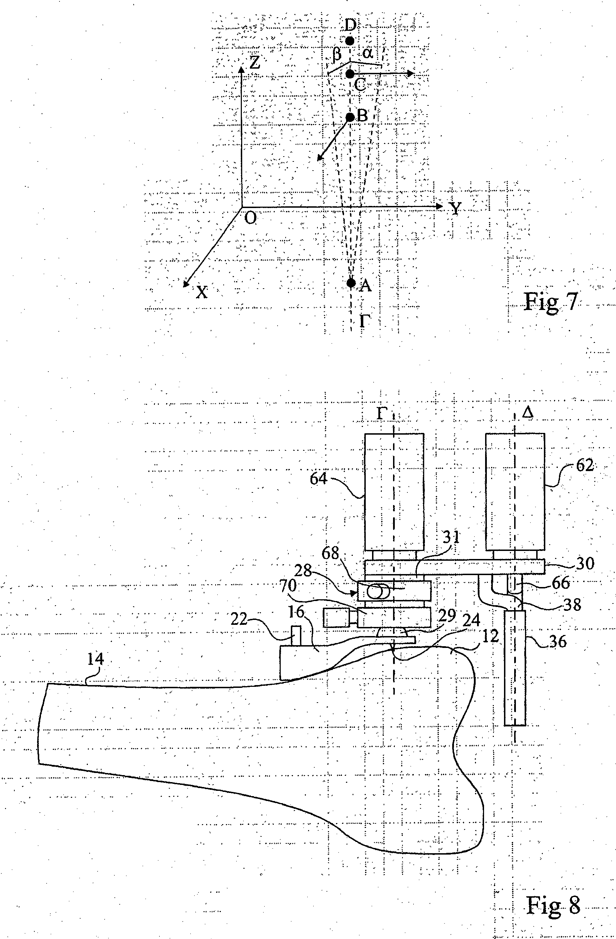

La figure 7 illustre, plus en détail, un exemple de fonctionnement du système d'ajustement 28. L'axe de rotation Γ est défini par les points A et D. A titre d'exemple, dans le premier exemple de réalisation, le point A correspond au centre du joint sphérique 34 et le point D correspond au centre de la liaison à pivot 31. Les points B et C se trouvent sur l'axe de rotation Γ et correspondent respectivement au centre du moyen de réglage 70 et du moyen de réglage 68. On définit un repère orthogonal, dans lequel l'axe (OZ) correspond à la position initiale de l'axe de rotation Γ.

A partir d'une position initiale, le moyen de réglage 70 permet de déplacer le point B selon la direction (OX) dans le sens positif ou négatif, et le moyen de réglage 68 permet de déplacer le point C selon la direction (OY) dans le sens positif ou négatif. L'ajustement de la position de l'axe de rotation Γ est donc obtenu en déplaçant les points B et C qui entraînent l'axe dé rotation Γ. Un déplacement du point B selon la direction (OX) d'une distance donnée entraîne une inclinaison de l'axe de rotation Γ autour de l'axe (OY) d'un angle β et un déplacement du point C selon la direction (OY) d'une distance donnée entraîne une inclinaison de l'axe de rotation Γ autour de l'axe (OX) d'un angle α.

Chaque moyen de réglage 68, 70 peut être constitué d'un chariot se déplaçant dans un rail s'étendant selon la direction (OX) ou la direction (OY), le chariot étant actionné par une vis. Il est à noter que, si les plages d'ajustement sont suffisamment importantes de sorte que les angles α et β puissent dépasser 1°, il est préférable de permettre aux points B et C de se déplacer le long de l'axe de rotation Γ pour s'adapter au changement de longueur résultant du déplacement linéaire lors des ajustements. Ceci peut être obtenu en utilisant des rails en arc de cercle.

La figure 8 représente un troisième exemple de réalisation du dispositif selon l'invention. Les éléments communs au premier exemple de réalisation sont indiqués par des références identiques. Le troisième exemple de réalisation consiste à ne conserver qu'une partie du dispositif selon le premier exemple de réalisation. Plus précisément, le dispositif selon le troisième exemple de réalisation est constitué, de l'embase 16, du système d'ajustement 28, du bras 30 et du guide 36. Les axes de rotation Γ et Δ sont de préférence maintenus sensiblement parallèles l'un à l'autre et le guide 36 s'étend de préférence au moins en partie transversalement à la tête de l'os. A titre d'exemple, le guide 36 est représenté en figure 8 avec une longueur inférieure à la longueur du guide de la figure 2. Le fonctionnement du dispositif selon le troisième exemple de réalisation est similaire à celui du dispositif selon le premier exemple de réalisation. Le dispositif selon le troisième exemple de réalisation est, par exemple, adapté à la réalisation de coupes au niveau d'un seul condyle du genou.

Selon une variante de l'invention, le système d'ajustement 28 est commandé par des moteurs électriques. Le réglage précis de la position de l'axe de rotation Δ par rapport à la tête 12 de l'os 14 peut alors être assisté par ordinateur.

Le dispositif de guidage de coupe selon la présente invention comporte de nombreux avantages :
Premièrement, le dispositif selon l'invention occupe une volume réduit, et donc ne nécessite pas de modifier techniques opératoires classiques, notamment en ce qui concerne les incisions à réaliser pour la mise en place du dispositif.
Deuxièmement, le dispositif de guidage de coupe permet de réaliser plusieurs plans de coupe distincts au niveau de la tête d'un os au moyen d'un seul dispositif de guide de coupe.
Troisièmement, le dispositif de guide de coupe peut facilement être adapté à différents outils de coupe tels que des fraises de coupe ou des scies oscillantes, simplement en changeant le guide de coupe.
Quatrièmement, des moteurs électriques peuvent facilement être montés sur le dispositif de coupe, permettant une commande simple et précise du dispositif assistée par ordinateur.

Bien entendu, la présente invention est susceptible de diverses variantes et modifications qui apparaîtront à l'homme de l'art. En particulier, le corps rigide 76 peut être fixé temporairement à l'élément de liaison 72 en prévoyant, par exemple, une fente au niveau de l'élément de liaison 72 dans laquelle est insérée temporairement une partie correspondante d'un corps rigide.

## Revendications

1. Système de guidage (10) d'un outil de coupe (44) adapté à couper des portions osseuses au niveau de la tête (12) d'un os (14), comprenant :
- un dispositif comportant une embase (16, 18) destinée à être fixée au côté de l'os au niveau de ladite tête ;
- un moyen d'ajustement (28) de l'inclinaison d'un premier axe de rotation (Γ) par rapport à ladite embase autour de deux axes (OX,OY) sensiblement perpendiculaires ;
- un bras (30, 32), une extrémité dudit bras étant montée pivotante sur ladite embase selon le premier axe de rotation ; et
- un guide (36) destiné à porter l'outil,
**caractérisé en ce que**:
le guide est monté pivotant sur le bras selon un second axe de rotation (Δ) ; et **en ce que** le système comprend
- un premier actionneur (64) porté par le bras, couplé audit bras et adapté à déplacer ce bras (30, 31) de façon assistée par ordinateur autour du premier axe de rotation (Γ) ; et
- un second actionneur (62) porté par le bras, couplé audit guide et adapté à déplacer le guide (36) de façon assistée par ordinateur autour du second axe de rotation (Δ), dans lequel les premier et deuxième actionneurs sont espacés l'un de l'autre d'une distance fixe dans toutes les positions de fonctionnement du système, la distance entre les premier et deuxième axes de rotation étant fixes dans toutes les positions de fonctionnement du système.

2. Système selon la revendication 1, dans lequel l'embase du dispositif est liée au côté de l'os par au moins une broche.

3. Système selon la revendication 1, dans lequel le guide du dispositif comprend une fente plane délimitée par des parois planes supérieure et inférieure dans laquelle l'outil de coupe est destiné à être inséré.

4. Système selon la revendication 1, dans lequel l'outil de coupe est une scie oscillante.

5. Système selon la revendication 1, dans lequel l'outil de coupe est une fraise.

6. Système selon la revendication 1, dans lequel le bras du dispositif est connecté à l'embase par le moyen d'ajustement (28), le moyen d'ajustement étant agencé entre l'embase et le bras et pouvant modifier la position du bras par rapport à l'embase de sorte que l'inclinaison des premier et second axes de rotation est réglée autour de deux axes sensiblement perpendiculaires.

7. Système selon la revendication 1, dans lequel un corps rigide est fixé à un élément de liaison du dispositif, le corps rigide pouvant être localisé par un système de localisation apte à déterminer la position du dispositif dans l'espace.

8. Système selon la revendication 7, dans lequel le corps rigide (76) comprend des facettes rétro-refléchissantes (78), le système de localisation comprenant une source émettant un rayonnement infrarouge et plusieurs capteurs mesurant le rayonnement infrarouge réfléchi par les facettes rétro-réfléchissantes.

9. Système selon la revendication 7, dans lequel le réglage de l'inclinaison du premier axe de rotation est réalisé par une commande assistée par ordinateur.

10. Système selon la revendication 9, dans lequel les positions théorique et réelle de l'axe de rotation atteintes par le corps rigide et le système de localisation sont affichées sur un écran d'affichage.

11. Système selon la revendication 7, dans lequel le système de localisation est à base de technologie optique, magnétique ou ultrasonore.

12. Système selon la revendication 1, dans lequel le premier axe de rotation (Γ) et le second axe de rotation (Δ) sont parallèles.

## Patentansprüche

1. Ein System (10) zur Führung eines Schneidwerkzeugs (44) welches in der Lage ist, Knochenteile auf der Höhe des Kopfes (12) eines Knochens (14) zu schneiden, wobei das System Folgendes aufweist:
- eine Vorrichtung, die einen Sitz (16, 18) aufweist, vorgesehen zur Befestigung an der Seite des Knochens auf der Höhe des Kopfes;
- Mittel (28) zum Einstellen der Neigung einer ersten Drehachse (Γ) bezüglich des erwähnten Sitzes um zwei im Wesentlichen senkrechte Achsen (OX, OY);
- einen Arm (30, 32), wobei ein Ende des erwähnten Arms schwenkbar auf dem erwähnten Sitz entsprechend zur ersten Drehachse zusammengebaut ist; und
- eine Führung (36), vorgesehen um das Werkzeug zu tragen,
**dadurch gekennzeichnet, dass**:
- die Führung schwenkbar zusammengebaut ist auf dem Arm entsprechend einer zweiten Drehachse (Δ); und **dadurch gekennzeichnet, dass** das System Folgendes aufweist:
- einen ersten Aktor bzw. Betätiger (64), getragen durch den Arm, gekuppelt mit dem Arm und geeignet zur Anordnung dieses Arms (30, 31) in einer computerunterstützten Weise, um die erste Drehachse (Γ) herum; und
- einen zweiten Aktor (Betätiger) (62), getragen durch den Arm, gekuppelt mit der erwähnten Führung und geeignet um die Führung (36) in einer computerunterstützten Weise um die zweite Drehachse (Δ) zu versetzen, wobei die ersten und zweiten Aktoren voneinander um einen festen Abstand in allen Betriebspositionen des Systems beabstandet sind, wobei der Abstand zwischen den ersten und zweiten Drehachsen in allen Betriebspositionen des Systems festliegt.

2. Das System nach Anspruch 1, wobei der Sitz der Vorrichtung an der Seite des Knochens mit mindestens einem Stift befestigt ist.

3. Das System nach Anspruch 1, wobei die Führung der Vorrichtung einen ebenen Schlitz aufweist, begrenzt durch obere und untere ebene Stirnflächen, in die das Schneidwerkzeug geeignet ist eingesetzt zu werden.

4. Das System nach Anspruch 1, wobei das Schneidwerkzeug eine Oszillationssäge ist.

5. Das System nach Anspruch 1, wobei das Schneidwerkzeug eine Fräse ist.

6. Das System nach Anspruch 1, wobei der Arm der Vorrichtung mit dem Sitz durch die Einstellmittel (28) verbunden ist, wobei die Einstellmittel zwischen dem Sitz und dem Arm angeordnet und in der Lage sind, die Position des Armes bezüglich des Sitzes derart zu modifizieren, dass die Neigung der ersten und zweiten Rotationsachsen um zwei im Wesentlichen senkrechte Achsen eingestellt wird.

7. Das System nach Anspruch 1, wobei ein starrer Körper an einem Verbindungselement der Vorrichtung befestigt ist, wobei der starre Körper durch ein Lokalisierungssystem, geeignet zur Bestimmung der räumlichen Position der Vorrichtung, lokalisiert ist.

8. Das System nach Anspruch 7, wobei der starre Körper (76) retroreflektierende Facetten (78) aufweist, wobei das Lokalisierungssystem eine Infrarot-Strahlung emittierende Quelle und eine Vielzahl von Sensoren aufweist, welche die Infrarot-Strahlung reflektiert durch die retroreflektierenden Facetten misst.

9. Das System nach Anspruch 7, wobei die Einstellung der Neigung der ersten Drehachse durch eine computerunterstützte Steuerung ausgeführt wird.

10. Das System nach Anspruch 9, wobei theoretische und tatsächliche Positionen der Drehachse, erreicht durch den starren Körper und das Lokalisierungssystem, auf einem Anzeigeschirm angezeigt werden.

11. Das System nach Anspruch 7, wobei das Lokalisierungssystem durch optische, magnetische oder Ultraschall-Technologie ausgeführt ist.

12. Das System nach Anspruch 1, wobei die erste Drehachse (Γ) und die zweite Drehachse (Δ) parallel verlaufen.

## Claims

1. A system (10) for guiding a cutting tool (44) capable of cutting bone portions at the level of the head (12) of a bone (14), comprising:
- a device comprising a seat (16, 18) intended to be fastened to the side of the bone at the level of said head;
- means (28) for adjusting the inclination of a first rotation axis (Γ) with respect to said seat around two substantially perpendicular axes (OX, OY);
- an arm (30, 32), one end of said arm being pivotally assembled on said seat according to the first rotation axis; and
- a guide (36) intended to support the tool,
**characterized in that**:
- the guide is pivotally assembled on the arm according to a second rotation axis (Δ); and **in that** the system comprises:
- a first actuator (64) supported by the arm, coupled to said arm and adapted to displace this arm (30, 31) in a computer assisted manner around the first rotation axis (Γ); and
- a second actuator (62) supported by the arm, coupled to said guide and adapted to displace the guide (36) in a computer assisted manner around the second rotation axis (Δ), wherein the first and second actuators are spaced from one another by a fixed distance in all the operating positions of the system, the distance between the first and second rotation axes being fixed in all the operating positions of the system.

2. The system of claim 1, wherein the seat of the device is fastened to the side of the bone by at least one pin.

3. The system of claim 1, wherein the guide of the device comprising a planar slot delimited by upper and lower planar faces in which the cutting tool is adapted to be inserted.

4. The system of claim 1, wherein the cutting tool is an oscillating saw.

5. The system of claim 1, wherein the cutting tool is a mill.

6. The system of claim 1, wherein the arm of the device is connected to the seat by the adjusting means (28), the adjusting means being arranged between the seat and the arm and being capable of modifying the position of the arm with respect to the seat so that the inclination of the first and second rotation axes is adjusted around two substantially perpendicular axes.

7. The system of claim 1, wherein a rigid body is fixed to a linking element of the device, the rigid body being localized by a localization system adapted to determine the spatial position of the device.

8. The system of claim 7, wherein the rigid body (76) comprises retro-reflecting facets (78), the localization system comprising a source emitting an infrared radiation and a plurality of sensors measuring the infrared radiation reflected by the retro-reflecting facets.

9. The system of claim 7, wherein the adjustment of the inclination of the first rotation axis is carried out by a computer assisted control.

10. The system of claim 9, wherein theoretical and actual positions of the rotation axis attained by the rigid body and the localization system are displayed on a display screen.

11. The system of claim 7, wherein the localization system is carried out by an optical, magnetic or ultrasonic technology.

12. The system of claim 1, wherein the first rotation axis (Γ) and the second rotation axis (Δ) are parallel.
